# EUROPEAN PATENT SPECIFICATION

(11) **EP 1 313 379 B1**
(45) Date of publication and mention of the grant of the patent: **30.06.2004**
(21) Application number: 01969521.2
(22) Date of filing: 26.07.2001
(51) Int. Cl.: A23L 2/52

(54) **ALPHA-(1,4)LINKED GLUCOSE POLYMERS CONTAINING ORAL COMPOSITIONS TO ALLEVIATE OR PREVENT TOOTH DAMAGE**
ALPHA-(1,4) GLUKOSE-POLYMER ENTHALTENDE ORAL-ZUSAMMENSETZUNGEN ZUR LINDERUNG ODER VERHINDERUNG VON ZAHNSCHÄDEN
NOUVELLE COMPOSITION & SON UTILISATION

(30) Priority: 01.08.2000 GB 0018849
(43) Date of publication of application: 28.05.2003
(73) Proprietor: SMITHKLINE BEECHAM PLC, Brentford, Middlesex TW8 9GS (GB)
(72) Inventor: PARKER, David Myatt, Coleford, Gloucestershire GL16 8JB (GB); ROEDIG-PENMAN, Andrea, Coleford, Gloucestershire GL16 8JB (GB)
(74) Representative: White, Susan Mary
(86) International application number: PCT/EP2001/008638
(87) International publication number: WO 2002/009537

(56) References cited:
- EP-A- 0 264 117
- WO-A-00/13531
- US-A- 4 853 237

## Description

The present invention relates to carbohydrate-containing compositions for oral use, such as beverages and confectionery compositions, and to the use of α-(1,4)-linked glucose polymers in such compositions to alleviate or prevent the tooth damage associated with the consumption of sugars.

Dental caries and dental erosion are caused by the action of acids on the enamel of the tooth surface. Dental erosion is typically associated with the direct consumption of acids such as fruit acids whilst dental caries is associated with the consumption of sugars. The acid which gives rise to dental caries is produced by fermentation of sugars by oral plaque bacteria covering the enamel surface. A particular problem arises with frequent consumption of products containing carbohydrates serving as a source of energy eg. so-called energy or sports drinks. The most common source of carbohydrate in oral products for conferring energy, such as sports drinks, are mono-and di-saccharides such as for example glucose (dextrose), sucrose and maltose. Longer chain polymers of glucose such as maltodextrins are also employed in such products as a source of energy. Mono- and di-saccharides and maltodextrins have been found in a rat model of the disease to be readily fermentable by plaque bacteria to produce acids (Grenby and Mistry, 1996, Caries Research **30**, 289). The acid produced by the fermentation of sugars by oral plaque bacteria reduces the pH of plaque fluid. As the pH is reduced, the plaque fluid becomes less saturated with respect to calcium hydroxyapatite, the mineral constituent of enamel. The 'critical pH' below which the plaque fluid becomes unsaturated with respect to apatite is considered to be around 5.5. This is dependent upon the individual's saliva composition and the site within the mouth. (Meurman and ten Cate, 1996 Eur J Oral Sci 104, 199-206).

Maltodextrins are carbohydrates which are also known as glucose polymers. They are usually derived from starch, for example corn starch, by hydrolysis. They largely comprise polymers of three or more dextrose units in length but also contain a small percentage, typically up to about 10 % by weight, of monosaccharides or disaccharides. The preparation of maltodextrin from starch results in a range of polymer chain lengths. The degree of depolyinerisation of starch is expressed as the dextrose equivalent (D.E.) which is the amount of total reducing sugars present, expressed as dextrose and calculated as a percentage of the total dry matter. Glucose (dextrose) has a D.E. of 100. Glucose syrups generally have a D.E. of 20 or more whereas maltodextrins generally have a D.E. of less than 20. The higher the D.E. value the greater will be the quantity of reducing sugars it contains and hence the more readily the carbohydrate source will be fermented by oral bacteria. Maltodextrins having D.E. values in the range 1 to 20 are commercially available with low % content of mono- and di-saccharides as detailed below.

Commercial hydrolysis of starch may be controlled to provide maltodextrins varying in D.E. and with a low percentage content of mono- and di-saccharides. For example Cerestar (Trafford Park, Manchester M17 1PA, UK) offer a range of maltodextrins with D.E. of from 5 to 18.5 and Staley (A.E. Staley Manufacturing Company, 2200 E.Eldorado Street, Decatur, IL 62525 USA) offer maltodextrins with D.E. of 1 to 18. Low D.E. glucose syrups with restricted % content of mono- and di-saccharides are also commercially available.

| | Saccharide distribution (%) | | | |
|---|---|---|---|---|
| | Mono- | Di- | Tri- | Higher |
| Staley Star-Dri Maltodextrin 5 (D.E. 5) | 0.9 | 0.9 | 1.0 | 97.2 |
| Staley Star-Dri Maltodextrin 10 (D.E. 10) | 0.6 | 2.8 | 4.4 | 92.2 |
| Staley Star-Dri Maltodextrin 15 (D.E. 15) | 1.3 | 4.1 | 6.0 | 88.6 |
| Cerestar C-Pur 01910 Maltodextrin (D.E. 14) | 1 | 3 | 6 | 90 |
| Cerestar C-Sweet 01411 Glucose Syrup(D.E.29) | 4 | 11 | 16.5 | 68.5 |

Polysaccharide sources of carbohydrate such as maltodextrins and glucose syrups are rapidly converted to glucose in the mouth by the action of the enzyme alpha-amylase. The alpha amylase enzyme hydrolyses the α-(1,4) linkages of non-cariogenic polysaccharides to form cariogenic monosaccharides and disaccharides such as glucose and maltose. Although there is some evidence for the presence of alpha-amylase-producing bacteria in dental plaque, the majority of alpha-amylase activity is salivary in origin (Scannapieco et al, 1993, Critical Reviews in Oral Biology and Medicine 4, 301-307).

The α-amylase enzyme is able to convert essentially non-cariogenic long chain polymers of glucose into cariogenic substrates that may then be metabolised by plaque bacteria, producing organic acid as a by-product. The cariogenic potential of maltodextrins has been evaluated in a human model by Al-Khatib et al, 1997, Caries Research 31, 316, abstracts 106 & 107. Maltodextrins were found to possess a lower acidogenic potential than sucrose but were found to have demineralising activity in an intra-oral cariogenicity test.

There is accordingly a general consensus in the literature that maltodextrins as well as sugars are disadvantageous to the dentition.

European Patent Application EP 0 264 117 addresses the problem of providing a fitness drink which will maintain blood glucose levels in blood during physical exercise, replace lost body fluids and salts and also inhibit damage to the dentition caused by fermentable carbohydrate. EP 0 264 117 describes fitness drink powder compositions comprising 60 to 85 % by weight long chain glucose polymers as the source of carbohydrate with the pH of the composition regulated between pH5.2 and 5.8. According to EP 0 264 117, the long chain glucose polymer preferably contains less than 10% by weight of monosaccharides and disaccharides. However no evidence for any effect on dentition is presented and it can be predicted that salivary amylase will produce fermentable sugars from such a composition.

Swedish Patent publication SE 8904190 discloses a composition intended for oral consumption for use in energy-requiring physical activity comprising maltodextrin as the main energy source and supplemented with xylitol as a caries-preventing substance. SE 8904190 addresses the problem of providing a slowly absorbed drink product based on low molecular weight carbohydrate sources such as dextrose and sucrose and of caries formation due to use of these sources of carbohydrate as a substrate for the bacterial flora in the mouth. The maltodextrin composition of SE 8904190 is defined in terms of its mono-, di- and oligosaccharide content up to 10 glucose units in length with the remainder (55 to 70% by weight) being oligosaccharides of over 10 glucose units in length. The range for monosaccharide and disaccharide content is from 2.1 to 4.0 % by weight. The preferred monosaccharide and disaccharide content of the maltodextrin composition is 3.0% by weight. The pH of the compositions of SE 8904190 is not defined. Whilst SE 8904190 states that the carbohydrate source should not be a good substrate for caries-producing bacteria, it is notable that the only example in the specification, a sports drink composition, contains 51.8% by weight maltodextrin and 38% by weight of the cariogenic monosaccharide fructose. Due to the action of α-amylase and of oral bacteria, the compositions disclosed in SE 8904190 will inevitably have the potential for plaque acid production and tooth demineralisation.

The present invention provides non-cariogenic, carbohydrate-containing compositions for oral administration comprising α-(1,4)-linked polymers of glucose such as maltodextrin as the primary source of carbohydrate. Use of such compositions according to the present invention will overcome the problem of the potential damage to the teeth caused by plaque acid produced in the mouth by oral bacteria. For the avoidance of doubt, reference herein to α-(1,4)-linked polymers of glucose includes polymers having α-(1,6) linkages as well as α-(1,4) linkages.

It has now been discovered that plaque acid production can be inhibited by using compositions formulated at low pH with α-(1,4)-linked polymers of glucose such as maltodextrin as the primary carbohydrate source. Whilst not being bound by theory, it is postulated that at a reduced pH, the α-amylase enzyme is not able to hydrolyse the α-(1,4) linkage and convert the glucose polymer into the readily fermentable mono- and di-saccharides. Therefore compositions may be formulated to contain energy-producing carbohydrate with minimal damage to teeth from plaque acid production.

According to the present invention there is provided the use of a carbohydrate-containing composition having an effective pH of 4.5 or less and comprising at least 1.0% by weight of an α-amylase digestible, α-(1,4)-linked polymer of glucose as a source of carbohydrate, in which composition the concentration of mono- and di-saccharides is no greater than 2.0% by weight, in the manufacture of an orally administrable composition for the reduction or prevention of tooth damage by plaque acid production.

In the context of the present invention, effective pH is defined as the pH of a composition that will confer a transient intra-oral pH of 4.5 or less during administration of the composition whilst it is in contact with saliva in the mouth. Compositions formulated to confer a pH below pH 4.5 have been found effective and for greatest benefit the effective pH should be below 4.0. Typically compositions according to the invention will have an effective pH no less than 2.0.

The carbohydrate source for use in the present invention will suitably be a maltodextrin having a low DE, typically 15 or less, such that the concentration of mono- and di-saccharides is minimised. There is no particular upper limit to the concentration of carbohydrate to be applied to the composition other than that dictated by the practicalities of preparation and other organoleptic considerations, provided that the concentration of mono- and disaccharides in the composition is minimised. As an approximate guide, the concentration of mono- and di-saccharides in the composition will preferably be no greater than 1.5% by weight and more advantageously no greater than 1.0% or even 0.5% by weight.

The invention is applicable to a wide range of carbohydrate-containing products for oral consumption or use, in particular to beverages and confectionary products. Compositions may be in the form of liquids, solids or semi-solids. The term beverage encompasses ready to drink liquid compositions as well as concentrates and powder formulations for dilution or dissolution. The invention may be applied in a variety of beverages such as concentrates, still or carbonated drinks with or without fruit juices or fruit extracts, and in particular to drinks such as sport and energy drinks or vitamin added beverages.

Compositions may be unsweetened or sweetened with intense sweeteners such as saccharine, aspartyl phenyl alanyl methyl ester, or other non-sugar sweeteners known in the art. Compositions may also contain other conventional additives such as sodium benzoate, sorbic acid, sodium metabisulfite, ascorbic acid, flavourings, colourings, stabilizers, eg. food hydrocolloids and carbon dioxide.

The present invention is particularly suitable for use in sport drinks formulated with about 6% carbohydrate, for example in the range 4.0 to 8.0% carbohydrate, and in energy providing products made with higher levels of carbohydrate, eg. about 15 to 25% carbohydrate. If a fruit juice or similar substance containing fermentable, mono- or disaccharide carbohydrate sources is a component of the composition then this will contribute to the concentration of mono- and disaccharides in the composition and appropriate allowance will be required.

High energy compositions formulated in accordance with the present invention containing α-(1,4)-linked polymers of glucose as the primary source of carbohydrate energy, for example compositions having more than about 15% by weight carbohydrate, in particular more than 20% by weight carbohydrate, are believed to be novel and as such form part of the present invention.

The introduction of acidic components *per se* to the composition is itself potentially disadvantageous in view of the potential for dental erosion thought to be caused *inter alia* by acidic foodstuffs leaching out calcium from the teeth faster than it can be replaced by normal remineralisation processes. Lussi et al (1995, Caries Res 29, 349-354) associated the pH and titratable acidity of a beverage with its erosive potential; the greater the concentration of acid in the beverage the more damaging to teeth it became.

There are methods known in the art to mitigate the erosive potential of food acidulants. WO 92/05711 discloses a method for preventing the erosion of tooth enamel by consuming an acid beverage (having a pH of less than 5.5) comprising from 0.02% to 0.15% of calcium in the form of a calcium citrate malate complex having a molar ratio of citrate to malate of 1:0.5 to 1:4.5. WO 97/30601 and WO 99/08550 disclose compositions having reduced tooth erosion properties containing a calcium compound and an acidulant characterised in that calcium is present in the range of 0.3 to 0.8 mol per mol of acidulant and the pH of the composition is from 3.5 to 4.5. WO 00/13531 discloses the use of viscosity modifying polymer materials, commonly used as thickening agents, stabilisers and emulsifyers, in acidic compositions for oral use to alleviate or inhibit the tooth damage associated with the consumption of acid.

When used in conjunction with known methods for controlling dental erosion based on addition of calcium and/or viscosity modifying polymer material, the present invention is particularly suitable for application to acidic, carbohydrate-containing products for oral consumption such as acidic sports and energy beverages, acidic beverages made with fruit juices and also to other acidic products to be taken orally. The teaching of the above-mentioned references is accordingly incorporated by reference.

Acid compositions may contain organic and/or inorganic acids and may be supplemented with vitamins such as for example B vitamins and ascorbic acid. Acid solutions may also contain sodium ions, particularly in the formulation of sport drinks. Preferred acidulants include potable acids such as citric, malic, lactic, phosphoric, acetic and tartaric acids. The invention is advantageously applied to drink products containing natural or added citric acid. The acidulant concentration in a composition will be determined by the type of product, the desired effective pH, the desired organoleptic properties and the acidity of the chosen acid source. The acidity of a composition may be expressed in terms of titratable acidity which is a measure of the percentage weight of acid present in a solution as calculated from the volume of sodium hydroxide required to neutralise the acidic species present. In practice, titratable acidity is measured potentiometrically with standardised sodium hydroxide solution of a known concentration at a temperature of 20 degrees Centigrade. A typical beverage will have a titratable acidity in the range 0.01 to 4%w/w and a typical fruit-flavour ready to drink beverage will have a titratable acidity in the range 0.1 to 2%w/w. Typically the acid concentration in compositions of the invention, for example the acid concentration in a fruit-flavour product would be in the range 0.01% w/w to 4% w/w, suitably in the range 0.1% w/w to 2.5% w/w. A typical ready to drink fruit-flavoured beverage based on citric and/or malic acid as the acidulant will have an acid concentration in the range 0.01 to as great as 2% w.w, preferably 0.01 to 1.0 %w/w of the beverage composition. In a concentrate for dilution, typical citric/malic acid concentration will be in the range 0.1 to 4%w/w of the composition. Mixtures of potable acids may be used, for example mixtures of acids selected from citric, malic, phosphoric and lactic acids and other suitable food grade excipients known in the art.

The effective pH of compositions according to the invention will vary according to type of product, acid content and desired organoleptic properties. A typical effective pH range of compositions is from pH 2.4 to pH 4.0, and more preferably from pH 2.7 to pH 4.0, especially for beverages containing fruit acids. It will be appreciated that for liquid compositions such as beverages, the effective pH will be very close to the actual pH of the composition.

Compositions according to the invention may be prepared by mixing the ingredients according to conventional methods. Solid ingredients may be dissolved in water or in hot water if required prior to mixing with other components. Typically beverage compositions are pasteurised prior to filling in bottles or cans or other packs or are "in-pack pasteurised" after filling.

The invention is illustrated by the following Examples:

### Example 1 - Effect of pH on Alpha Amylase Hydrolysis of 14DE Maltodextrin

To test the invention that a reduced pH will inhibit salivary alpha amylase ability to hydrolyse the α-(1,4) linkage of glucose polymers, 14DE maltodextrin solutions as detailed below were incubated at 37°C with and without salivary alpha amylase. Amylase was purchased from Sigma-Aldrich Company Ltd, Poole, Dorset, UK. One unit of alpha amylase activity is defined as the quantity that will liberate 1.0 mg of maltose from starch in 3 minutes at pH6.9 at 20 degrees centigrade. The pH of the incubations was adjusted by the addition of sodium hydroxide / hydrochloric acid. Samples were withdrawn from the incubation immediately after addition of the enzyme (time 0) and after 3 and 10 minutes. These were immediately diluted 1/200 in 0.1M sodium hydroxide.

| **Composition of solution** | |
|---|---|
| 14DE Maltodextrin (Cerestar C-Pur 01910) | 10% w/v |
| Sodium chloride | 0.1% w/v |
| Citric Acid | 20 mmolar |
| Human α amylase (Sigma type XIII-A) | 25 units per ml |

### Results

The composition of the carbohydrate species in the maltodextrin / enzyme incubations was subsequently established by HPLC.

HPLC details were as follows:

| | |
|---|---|
| Column | DIONEX column, CarboPac PA-100 |
| Temperature | 25°C |
| Flow rate | 1.0ml/minute |
| Run Time | 30 minutes |
| Mobile Phase | 100% 0.1M NaOH to 100% 0.1M NaOH0.25M sodium acetate |

Results are described as the % of a carbohydrate species as part of the total carbohydrate.

| **Time: 0 minutes** | | | | | | | |
|---|---|---|---|---|---|---|---|
| Composition | No enzyme pH 7.0 (in %) | With enzyme pH 7 (in %) | With enzyme pH 5 (in %) | With enzyme pH 4.5 (in %) | With enzyme pH 4 (in %) | With enzyme pH 3.5 (in %) | With enzyme PH 3 (in %) |
| DP1 | 0.6 | 0.6 | 1.0 | 0.9 | 0.8 | 0.8 | 0.8 |
| DP2 | 2.0 | 2.2 | 0.9 | 0.9 | 1.0 | 1.5 | 1.8 |
| DP3 | 2.5 | 2.7 | 1.3 | 1.3 | 1.4 | 1.7 | 2.2 |
| DP4 | 1.7 | 2.0 | 1.5 | 1.4 | 1.4 | 1.5 | 1.8 |
| DP5 | 6.2 | 5.8 | 5.1 | 5.0 | 5.3 | 5.7 | 6.7 |
| DP6 | 6.5 | 5.8 | 4.1 | 4.2 | 4.5 | 5.0 | 6.2 |
| DP7 | 5.8 | 5.3 | 4.4 | 4.4 | 4.6 | 5.0 | 6.0 |
| Other | 74.8 | 75.6 | 81.5 | 81.9 | 80.9 | 78.8 | 74.5 |

| **Time: 3 minutes** | | | | | | | |
|---|---|---|---|---|---|---|---|
| Composition | No enzyme pH 7.0 (in %) | With enzyme pH 7 (in %) | With enzyme pH 5 (in %) | With enzyme pH 4.5 (in %) | With enzyme pH 4 (in %) | With enzyme pH 3.5 (in %) | With enzyme PH 3 (in %) |
| DP1 | 0.6 | 1.1 | 1.3 | 1.0 | 0.9 | 0.8 | 0.7 |
| DP2 | 1.9 | 3.9 | 1.2 | 1.1 | 1.1 | 1.2 | 1.5 |
| DP3 | 2.3 | 4.6 | 1.8 | 1.5 | 1.5 | 1.7 | 1.9 |
| DP4 | 1.6 | 2.3 | 1.7 | 1.6 | 1.5 | 1.5 | 1.5 |
| DP5 | 6.1 | 4.2 | 4.8 | 5.1 | 5.6 | 5.8 | 5.8 |
| DP6 | 6.3 | 3.5 | 3.6 | 4.1 | 4.8 | 5.1 | 5.5 |
| DP7 | 5.7 | 3.4 | 4.1 | 4.4 | 4.9 | 5.0 | 5.1 |
| Other | 75.4 | 77.1 | 81.6 | 81.1 | 79.6 | 78.9 | 78.2 |

| **Time: 10 minutes** | | | | | | | |
|---|---|---|---|---|---|---|---|
| Composition | No enzyme pH 7.0 (in %) | With enzyme pH 7 (in %) | With enzyme pH 5 (in %) | With enzyme pH 4.5 (in %) | With enzyme pH 4 (in %) | With enzyme pH 3.5 (in %) | With enzyme pH 3 (in %) |
| DP1 | 0.6 | 2.6 | 1.7 | 1.3 | 0.9 | 0.9 | 0.7 |
| DP2 | 1.8 | 6.4 | 1.7 | 1.2 | 1.1 | 1.4 | 1.5 |
| DP3 | 2.1 | 7.0 | 2.5 | 1.8 | 1.5 | 1.5 | 1.9 |
| DP4 | 1.5 | 3.3 | 1.9 | 1.7 | 1.5 | 1.5 | 1.5 |
| DP5 | 5.7 | 2.2 | 3.6 | 4.4 | 5.6 | 5.6 | 5.8 |
| DP6 | 5.8 | 0.9 | 2.4 | 3.3 | 5.2 | 4.8 | 5.4 |
| DP7 | 5.2 | 0.9 | 2.9 | 3.8 | 4.9 | 4.8 | 5.1 |
| Other | 77.5 | 76.6 | 83.4 | 82.6 | 79.4 | 79.5 | 78.0 |
| [DP means degree of polymerisation; DP1 represents monosaccharide, DP2 disaccharide etc. 'Other' means other carbohydrate species calculated by difference.] | | | | | | | |

A reduction in pH to 4.5 inhibits the hydrolysis of the α-(1,4) linkages. Above pH 4.5, there is a reduction in higher sugar polymers (DP>5) and increase in mono-, di- and tri saccharides (DP1-3). Considerable hydrolysis of the maltodextrin was observed at pH7.0

### Example 2 - Effect of pH on Alpha Amylase Hydrolysis of 5DE Maltodextrin

To test the invention that a reduced pH will inhibit salivary alpha amylase ability to hydrolyse the α-(1,4) linkage of glucose polymers, 5DE maltodextrin solutions as detailed below were incubated at 37°C with and without salivary alpha amylase in a manner similar to that described in Example 1. The pH was adjusted by the addition of sodium hydroxide / hydrochloric acid Samples were withdrawn from the incubation immediately after addition of the enzyme (time 0) and after 10 minutes. These were immediately diluted 1/200 in 0.1M sodium hydroxide.

| **Composition of solution** | |
|---|---|
| 5DE Maltodextrin (Staley Star-Dri) | 18.75% w/v (18% carbohydrate) |
| Sodium chloride | 0.1% w/v |
| Citric Acid | 20 mmolar |
| Human α amylase (Sigma type XIII-A) | 25 units per ml where added |

### Results

The composition of the carbohydrate species in the maltodextrin / enzyme incubations was subsequently established by HPLC. HPLC details as per example 1.
Results are described as the % of a carbohydrate species as part of the total carbohydrate.

| **Time: 0 minutes** | | | | | | |
|---|---|---|---|---|---|---|
| Composition | No enzyme pH 7.0 (in %) | With enzyme pH 7 (in %) | With enzyme pH 5 (in %) | With enzyme pH 4.5 (in %) | With enzyme pH 4 (in %) | With enzyme pH 3.5 (in %) |
| DP1 | 1.0 | 1.0 | 1.0 | 1.0 | 1.0 | 1.0 |
| DP2 | 0.9 | 0.8 | 0.7 | 0.6 | 0.6 | 0.6 |
| DP3 | 1.1 | 1.1 | 0.9 | 0.8 | 0.7 | 0.7 |
| DP4 | 1.2 | 1.1 | 1.0 | 0.9 | 0.8 | 0.8 |
| DP5 | 2.9 | 2.8 | 2.8 | 2.7 | 2.6 | 2.6 |
| DP6 | 3.5 | 2.9 | 2.9 | 2.7 | 2.6 | 2.5 |
| DP7 | 3.6 | 3.2 | 3.2 | 3.1 | 3.0 | . 3.0 |
| Other | 85.8 | 87.1 | 87.6 | 88.2 | 88.7 | 88.9 |

| **Time: 10 minutes** | | | | | | |
|---|---|---|---|---|---|---|
| Composition | No enzyme pH 7.0 (in %) | With enzyme pH 7 (in %) | With enzyme pH 5 (in %) | With enzyme pH 4.5 (in %) | With enzyme pH 4 (in %) | With enzyme pH 3.5 (in %) |
| DP1 | 0.9 | 2.7 | 1.5 | 1.2 | 1.0 | 1.0 |
| DP2 | 0.9 | 4.9 | 1.8 | 1.0 | 0.7 | 0.5 |
| DP3 | 1.0 | 6.1 | 2.6 | 1.4 | 0.8 | 0.6 |
| DP4 | 1.1 | 3.2 | 1.3 | 1.0 | 0.9 | 0.8 |
| DP5 | 2.9 | 2.6 | 2.8 | 2.7 | 2.7 | 2.5 |
| DP6 | 3.4 | 1.8 | 2.5 | 1.5 | 2.7 | 2.4 |
| DP7 | 3.5 | 2.0 | 2.9 | 3.0 | 3.0 | 2.9 |
| Other | 86.4 | 76.8 | 84.6 | 88.2 | 88.4 | 89.3 |
| [DP means degree of polymerisation; DP1 represents monosaccharide, DP2 disaccharide etc. 'Other' means other carbohydrate species calculated by difference.] | | | | | | |

A reduction in pH to 4.5 inhibits the hydrolysis of the α-(1,4) linkages. Above pH 4.5 there is a reduction in higher sugar polymers (DP>5) and increase in mono-, di- and tri saccharides (DP1-3). Again, substantial hydrolysis was observed at pH7.0.

### Example 3 - Sport Drink Composition

Sport drinks compositions were prepared according to the formula detailed below. Four different maltodextrins each having a D.E. ranging from 6-14 were added to give a carbohydrate concentration of 6.4% by weight. The total volume of each test composition was 1 litre and the pH was 3.8. The sodium concentration was about 55mg per 100mls.

| **Composition of the Sport Drinks** | **Weights (g) in 1 Litre** |
|---|---|
| Sodium hydrogen sulphate (50% solution) | 1.9105 ml |
| Citric Acid anhydrous | 3.0 |
| Colour Orange Emulsion 61.461 | 1.84 |
| Potassium Sorbate | 0.3 886 |
| Aspartame | 0.2215 |
| Acesulfame K | 0.0709 |
| Ascorbic Acid | 0.2336 |
| Tri-sodium citrate dihydrate | 1.300 |
| Orange Flavour 10174-34 | 0.270 |
| Cloudifier Emulsion 61.459 | 0.470 |
| Calcium Carbonate (Sturcal F) | 0.930 |
| Maltodextrin | 66.66 |
| Water | To 11 |

The composition of the four maltodextrins used was established by HPLC (see below).

| **Composition** | **Maltodextrin 1** **(in %) DE=6** | **Maltodextrin 2** **(in %) DE=10** | **Maltodextrin 3** **(in %) DE=14- 16** | **Maltodextrin 4** **(in %) DE=14** |
|---|---|---|---|---|
| DP1 | 0.8 | 1.0 | 1.1 | 0.3 |
| DP2 | 1.0 | 1.9 | 4.0 | 2.9 |
| DP3 | 1.3 | 2.9 | 4.7 | 3.1 |
| DP4 | 1.5 | 2.4 | 3.8 | 2.5 |
| DP5 | 1.5 | 2.4 | 4.0 | 2.6 |
| DP6 | 1.6 | 3.7 | 4.3 | 3.3 |
| DP7 | 1.9 | 4.4 | 4.6 | 3.6 |
| Other | 90.4 | 81.3 | 73.5 | 81.7 |
| [ DP means degree of polymerisation; DP1 represents monosaccharide, DP2 disaccharide etc. 'Other' means other carbohydrate species calculated by difference.] | | | | |

### Plaque pH Study

The maltodextrin-containing sport drinks were evaluated by means of a plaque pH study to assess the utility of the invention with respect to the ability of plaque bacteria to produce acid from the formulations. This involved 14 volunteers in a seven leg study that also included a blank (sports drink formulation without carbohydrate) and sucrose and sorbitol positive and negative control legs (10% solutions dissolved in water). On each test day, a sample of plaque was taken from the buccal surfaces of four sites of the subjects' teeth using a sterile stainless steel straight probe. This formed the baseline plaque sample (time 0). The sample was mixed with 20 microlitres of distilled water and the pH measured with a micro electrode. Subjects then rinsed their mouths thoroughly with 15ml of the sports drinks or of the controls for 1 minute. Subjects then swallowed the drink. The pH of the plaque was subsequently determined after 2 and 5 minutes and thereafter at 5 minute intervals up to 30 minutes. A sport drink formulation without any carbohydrate was used as a blank. Statistical analysis was performed after obtaining all the data (Turkey's Significant Difference Test and Splined Stephan Curves). The method has been described by Toumba and Duggal, 1999 (British Dental Journal 186, 626 - 629).

### Results

The following table shows that the pH of the four different maltodextrin-containing compositions never dropped below 6.15 whereas the pH of the sucrose control composition dropped to 5.42. The criteria of "toothfriendliness" is that the pH does not drop below a pH of 5.5, below which enamel may begin to be dissolved. The maltodextrin formulations did not cause a reduction in plaque pH to a level for enamel damage to occur. The sport drink formulation without carbohydrate and the sorbitol control composition reduced plaque pH less than the test solutions. Analysis of the data showed that there was a statistically significant difference between the pH drop from sucrose and the pH drop of the four maltodextrin-containing compositions. There was no difference between the four maltodextrin-containing compositions. The results demonstrate that a beverage can be formulated containing appreciable quantities of low D.E. maltodextrin that has no significant cariogenic potential.

| | Mean pH at Time (minutes) | | | | | | | | Minimum pH (at any time) | |
|---|---|---|---|---|---|---|---|---|---|---|
| | 0 | 2 | 5 | 10 | 15 | 20 | 25 | 30 | Mean | SD |
| A | 6.91 | 6.91 | 6.46 | 6.43 | 6.57 | 6.73 | 6.86 | 6.86 | 6.20 | 0.342 |
| B | 6.91 | 6.91 | 6.33 | 6.47 | 6.61 | 6.61 | 6.89 | 6.86 | 6.26 | 0.342 |
| C | 6.98 | 6.98 | 6.48 | 6.50 | 6.69 | 6.72 | 6.84 | 6.94 | 6.28 | 0.393 |
| D | 6.95 | 6.95 | 6.20 | 6.46 | 6.50 | 6.60 | 6.77 | 6.91 | 6.15 | 0.335 |
| E | 6.86 | 6.86 | 6.68 | 6.90 | 6.96 | 7.02 | 7.08 | 6.94 | 6.61 | 0.299 |
| F | 6.86 | 6.86 | 6.13 | 5.67 | 5.73 | 5.99 | 6.39 | 6.34 | 5.42 | 0.181 |
| G | 6.96 | 6.96 | 7.02 | 6.96 | 7.00 | 6.89 | 6.93 | 6.96 | 6.73 | 0.348 |
| A: Maltodextrin 1 B: Maltodextrin 2 C: Maltodextrin 3 D: Maltodextrin 4 E: No maltodextrin F: 10% w/v sucrose G: 10% w/v sorbitol | | | | | | | | | | |

### Example 4 - Energy/Sport Drink Composition

Energy/sport drink compositions were prepared according to the formula detailed below. Three different 5 D.E. maltodextrin solutions were prepared using from 6-24% carbohydrate. The test compositions had a product acidity of 0.3% w/w citric acid monohydrate and a product pH was 3.2.

| Composition of the Energy Drinks (%w/v) | Solution 1(S1) | Solution 2(S2) | Solution 3(S3) |
|---|---|---|---|
| Citric Acid anhydrous | 0.262 | 0.262 | 0.262 |
| Potassium Sorbate | 0.03 | 0.03 | 0.03 |
| Sodium Benzoate | 0.0072 | 0.0072 | 0.0072 |
| Tri-sodium citrate dihydrate | 0.06 | 0.06 | 0.06 |
| Maltodextrin (Staley Star-Dri 5 D.E.) | 6.25 | 12.5 | 25.0 |
| Water | To 100 | To 100 | To 100 |
| Maltodextrin (Staley Star-Dri 5 D.E.) is 95% carbohydrate. | | | |

The composition of the three maltodextrin solutions used was established by HPLC (see below).

| Composition | Solution 1 (g/L) | Solution 2 (g/L) | Solution 3 (g/L) |
|---|---|---|---|
| DP1 | 0.766 | 1.555 | 1.840 |
| DP2 | 0.809 | 1.648 | 2.307 |
| [DP means degree of polymerisation; DP1 represents monosaccharide, DP2 disaccharide.] | | | |

### Plaque pH Study

The maltodextrin-containing energy/sport drinks were evaluated by means of a plaque pH study to assess the utility of the invention with respect to the ability of plaque bacteria to produce acid from the formulations. This was conducted in a similar manner to that described in Example 3. This involved 9 volunteers in a five leg study that also included acidified sucrose and acidified sorbitol positive and negative control legs (10% solutions dissolved in the same base composition as the test maltodextrin solutions). On each test day, a sample of plaque was taken from the buccal surfaces of the subjects' teeth using a sterile stainless steel straight probe. This formed the baseline plaque sample (time 0). The sample was mixed with 30 microlitres of distilled water and the pH measured with a micro electrode. Subjects then rinsed their mouths thoroughly with 15ml of the energy/sports drinks or of the controls for 1 minute. Subjects then swallowed the drink. The pH of the plaque was subsequently determined after 6 minutes and 40 seconds, 10 minutes, 15 minutes, 25 minutes and 30 minutes.

### Results

The following table shows that the pH of the three maltodextrin-containing compositions never dropped below 5.5 whereas the pH of the sucrose control composition dropped to 5.28. The criteria of "toothfriendliness" is that the pH does not drop below a pH of 5.5, below which enamel may begin to be dissolved. The maltodextrin formulations did not cause a reduction in plaque pH to a level for enamel damage to occur. The sorbitol control composition reduced plaque pH less than the test solutions.

| | Mean pH at Time (minutes:seconds) | | | | | |
|---|---|---|---|---|---|---|
| | 0 | 6:40 | 10 | 15 | 25 | 30 |
| S1 | 6.62 | 6.00 | 6.33 | 6.51 | 6.53 | 6.62 |
| S2 | 6.35 | 5.57 | 5.92 | 6.20 | 6.29 | 6.18 |
| S3 | 6.84 | 5.74 | 6.09 | 6.25 | 6.42 | .6.54 |
| S4 | 6.57 | 5.28 | 5.57 | 5.85 | 6.09 | 6.16 |
| S5 | 6.45 | 6.05 | 6.36 | 6.40 | 6.40 | 6.45 |
| [S4: Acidifed 10% w/v sucrose S5: Acidified 10% w/v sorbitol] | | | | | | |

The results demonstrate that a beverage can be formulated containing appreciable quantities of low D.E. maltodextrin that has minimal cariogenic potential.

### Example 5 - Powdered Sports Drink Composition

A powdered sport drink formulation was made according to the following list of ingredients that are dry blended typically using a ribbon blender until an homogeneous mixture is obtained. The product is then filled into appropriate packaging such as sachets, jars or drums.

| **Ingredients** | Kg |
|---|---|
| Maltodextrin (Cerestar C-Pur 01910) | 87.2 |
| Aspartame . | 0.2 |
| Acesulfame-k | 0.1 |
| Calcium carbonate | 1.24 |
| Citric acid anhydrous | 6.84 |
| Ascorbic acid | 0.32 |
| Trisodium citrate dihydrate | 2.83 |
| Orange flavour | 0.54 |
| Beta carotene (1% Cold Water Soluble) | 0.73 |
| Total | 100 |

75g of the powder was dissolved in water to a final volume of 1 litre to make an orange sport drink. The drink had a pH of approximately 4.

## Claims

1. The use of a carbohydrate-containing composition having an effective pH of 4.5 or less and comprising at least 1.0% by weight of an α-amylase digestible, α-(1,4)-linked polymer of glucose as a source of carbohydrate, in which composition the concentration of mono- and di-saccharides is no greater than 2.0% by weight, in the manufacture of an orally administrable composition for the reduction or prevention of tooth damage by plaque acid production.

2. Use of a composition according to claim 1 in which the α-(1,4)-linked polymer of glucose is a maltodextrin.

3. Use of a composition according to claim 2 in which the α-(1,4)-linked polymer of glucose is a maltodextrin having a dextrose equivalent (DE) of 15 or less.

4. Use of a composition according to any one of claims 1 to 3 in which the concentration of mono- and di-saccharides is no greater than 1.0% by weight.

5. Use of a composition according to any one of claims 1 to 4 having an effective pH in the range 2.0 to 4.0.

6. Use of a compostion according to any one of claims 1 to 5 which is a beverage.

7. Use of a compostion according to any one of claims 1 to 5 which is a confectionary product.

8. Use of a composition according to claim 6 which is a sport drink containing between 4.0 and 8.0% by weight carbohydrate.

9. Use of a composition according to claim 6 or 7 which is an energy product containing between 15 and 25% by weight carbohydrate.

10. Use of a composition according to claim 6 having an effective pH in the range 2.4 to 4.0.

11. Use of a composition according to any preceding claim further comprising calcium and/or a viscosity modifying material.

12. A carbohydrate-containing composition for oral administration having an effective pH of 4.5 or less and comprising at least 20.0% by weight of an α-amylase digestible, α-(1,4)-linked polymer of glucose as a source of carbohydrate, in which composition the concentration of mono- and di-saccharides is no greater than 2.0% by weight.

13. A composition according to claim 12 which is a beverage.

## Patentansprüche

1. Verwendung einer kohlehydrathaltigen Zusammensetzung mit einem effektiven pH von 4,5 oder weniger, die wenigstens 1,0 Gew.% eines α-Amylase-verdaubaren, α-(1,4)-verbundenen Polymers von Glucose als Kohlehydratquelle umfasst, worin die Konzentration von Mono- und Disacchariden in der Zusammensetzung nicht grösser als 2,0 Gew.% ist, in der Herstellung einer oral verabreichbaren Zusammensetzung zur Reduzierung oder Prävention von Zahnschäden durch Plaque-Säureproduktion.

2. Verwendung einer Zusammensetzung gemäss Anspruch 1, worin das α-(1,4)-verbundene Polymer von Glucose ein Maltodextrin ist.

3. Verwendung einer Zusammensetzung gemäss Anspruch 2, worin das α-(1,4)-verbundene Polymer von Glucose ein Maltodextrin mit einem Dextroseäquivalent (DE) von 15 oder weniger ist.

4. Verwendung einer Zusammensetzung gemäss einem der Ansprüche 1 bis 3, worin die Konzentration von Monound Disacchariden nicht grösser als 1,0 Gew.% ist.

5. Verwendung einer Zusammensetzung gemäss einem der Ansprüche 1 bis 4 mit einem effektiven pH im Bereich von 2,0 bis 4,0.

6. Verwendung einer Zusammensetzung gemäss einem der Ansprüche 1 bis 5, die ein Getränk ist.

7. Verwendung einer Zusammensetzung gemäss einem der Ansprüche 1 bis 5, die ein Konfekterzeugnis ist.

8. Verwendung einer Zusammensetzung gemäss Anspruch 6, die ein Sportgetränk ist, das zwischen 4,0 und 8,0 Gew.% Kohlehydrat enthält.

9. Verwendung einer Zusammensetzung gemäss Anspruch 6 oder 7, die ein Energieerzeugnis ist, das zwischen 15 und 25 Gew.% Kohlehydrat enthält.

10. Verwendung einer Zusammensetzung gemäss Anspruch 6 mit einem effektiven pH im Bereich von 2,4 bis 4,0.

11. Verwendung einer Zusammensetzung gemäss jedem vorhergehenden Anspruch, die ferner Calcium und/oder ein die Viskosität modifizierendes Material umfasst.

12. Kohlehydrathaltige Zusammensetzung zur oralen Verabreichung mit einem effektiven pH von 4,5 oder weniger, die wenigstens 20 Gew.% eines α-Amylaseverdaubaren, α-(1,4)-verbundenen Polymers von Glucose als Kohlehydratquelle umfasst, worin die Konzentration von Mono- und Disacchariden in der Zusammensetzung nicht grösser als 2,0 Gew.% ist.

13. Zusammensetzung gemäss Anspruch 12, die ein Getränk ist.

## Revendications

1. Utilisation d'une composition contenant des hydrates de carbone ayant un pH efficace de 4,5 ou moins et comprenant au moins 1,0 % en poids d'une α-amylase digestible, un polymère lié en α-(1,4) de glucose en tant que source d'hydrates de carbone, composition dans laquelle la concentration en mono- et di-saccharides n'est pas supérieure à 2,0 % en poids, dans la fabrication d'une composition susceptible d'être administrée par voie orale pour la réduction ou la prévention d'un endommagement des dents par la production d'une plaque acide.

2. Utilisation d'une composition selon la revendication 1, dans laquelle le polymère lié en α-(1,4) de glucose est une maltodextrine.

3. Utilisation d'une composition selon la revendication 2, dans laquelle le polymère lié en α-(1,4) de glucose est une maltodextrine ayant un équivalent de dextrose (DE) de 15 ou moins.

4. Utilisation d'une composition selon l'une quelconque des revendications 1 à 3, dans laquelle la concentration en mono- et di-saccharides n'est pas supérieure à 1,0 % en poids.

5. Utilisation d'une composition selon l'une quelconque des revendications 1 à 4, ayant un pH efficace dans la gamme de 2,0 à 4,0.

6. Utilisation d'une composition selon l'une quelconque des revendications 1 à 5, qui est une boisson.

7. Utilisation d'une composition selon l'une quelconque des revendications 1 à 5, qui est un produit de confiserie.

8. Utilisation d'une composition selon la revendication 6, qui est une boisson pour le sport contenant entre 4,0 et 8,0 % en poids d'hydrate de carbone.

9. Utilisation d'une composition selon la revendication 6 ou 7, qui est un produit énergétique contenant entre 15 et 25 % en poids d'hydrate de carbone.

10. Utilisation d'une composition selon la revendication 6, ayant un pH efficace dans la gamme de 2,4 à 4,0.

11. Utilisation d'une composition selon l'une quelconque des revendications précédentes, comprenant en outre du calcium et/ou une substance modifiant la viscosité.

12. Composition contenant des hydrates de carbone pour une administration orale ayant un pH efficace de 4,5 ou moins et comprenant au moins 20,0 % en poids d'une α-amylase digestible, un polymère lié en α-(1,4) de glucose en tant que source d'hydrates de carbone, composition dans laquelle la concentration en mono- et di-saccharides n'est pas supérieure à 2,0 % en poids.

13. Composition selon la revendication 12, qui est une boisson.
